# EUROPEAN PATENT APPLICATION

(11) **EP 4 714 429 A1**
(43) Date of publication of application: **25.03.2026**
(21) Application number: 24807582.2
(22) Date of filing: 16.05.2024
(51) Int. Cl.: A61K 8/85, A61K 8/02, A61K 8/44, A61K 8/36, A61K 8/34, A61Q 19/10

(54) **BIODEGRADABLE SCRUB PARTICLES AND SKIN CLEANSING COMPOSITION COMPRISING SAME**

(30) Priority: 17.05.2023 KR 20230063714
(71) Applicant: CJ Cheiljedang Corporation, Seoul 04560 (KR)
(72) Inventor: MUN, Bo Ra, Seoul 04560 (KR); LEE, Eun-Hye, Seoul 04560 (KR); YOON, Ki Chull, Seoul 04560 (KR)
(74) Representative: Meissner Bolte Partnerschaft mbB
(86) International application number: PCT/KR2024/006678
(87) International publication number: WO 2024/237711

(57) **Abstract**

The present invention relates to biodegradable scrub particles and a skin cleansing composition comprising same, wherein the biodegradable scrub particles comprise polyhydroxyalkanoate (PHA).

## Description

### Technical Field

The present disclosure relates to biodegradable scrub particles comprising a biodegradable polymer and to a skin cleansing composition comprising the biodegradable scrub particles.

### Background Art

Cleansing products commonly comprise a scrub agent for a variety of reasons, including removing dirt, waste products (e.g., sebum, dead skin cells, etc.), or cosmetics remaining on the skin. Such a scrub agent comprises particles prepared from petroleum-based synthetic resins such as polyethylene, polypropylene, and polystyrene. However, the petroleum-based synthetic resins would form microplastics and have a negative impact on the environment. Thus, there is a worldwide trend to ban the distribution of cleansing products that contain solid synthetic resin particles (microplastics) of 5 mm or less used for skin cleansing purposes. As a result, the need for cleaning products that contain natural raw material particles or biodegradable raw material particles is increasing.

In response to the above needs, cleaning products that contain natural raw material particles such as chitin derived from crustaceans or walnut shells have been developed; however, there is a problem with the high costs required in the production process. In addition, since the surface of natural raw material particles is relatively rough, there is also the problem of skin damage when the skin is cleansed with a cleansing product that contains such particles.

Accordingly, there is a need to develop a cleansing product that is less irritating to the skin, has excellent skin cleansing power, and is also biodegradable to be environmentally friendly.

### Disclosure of Invention

### Technical Problem

In order to solve the above problems of the prior art, an object of the present disclosure is to provide biodegradable scrub particles that are excellent in skin cleansing power while causing little irritation to the skin and, at the same time, are biodegradable to be environmentally friendly, and a skin cleansing composition comprising the biodegradable scrub particles.

### Solution to Problem

In order to accomplish the above object, the present disclosure provides biodegradable scrub particles, which comprise a polyhydroxyalkanoate (PHA) comprising a repeat unit derived from 4-hydroxybutyrate (4-HB) in an amount of 0.1 to 50% by weight based on the total weight of the polyhydroxyalkanoate (PHA), wherein the particle size (D₅₀) of the biodegradable scrub particles is 10 to 1,000 µm.

According to another embodiment of the present disclosure, the polyhydroxyalkanoate (PHA) may further comprise a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

According to another embodiment of the present disclosure, the polyhydroxyalkanoate (PHA) may further comprise a repeat unit derived from 3-hydroxybutyrate (3-HB).

According to another embodiment of the present disclosure, the particle size (D₅₀) of the biodegradable scrub particles may be 10 to 800 µm.

According to another embodiment of the present disclosure, the content of the repeat unit derived from 4-hydroxybutyrate (4-HB) may be 1 to 10% by weight based on the total weight of the polyhydroxyalkanoate (PHA).

Meanwhile, in order to accomplish the above object, the present disclosure provides a skin cleansing composition, which comprises the biodegradable scrub particles; a surfactant; and a polyol.

According to an embodiment of the present disclosure, the content of the biodegradable scrub particles may be 0.5 to 30% by weight based on the total weight of the skin cleansing composition.

According to another embodiment of the present disclosure, the content of the surfactant may be 10 to 40% by weight, and the content of the polyol may be 1 to 15% by weight, based on the total weight of the skin cleansing composition.

According to another embodiment of the present disclosure, the skin cleansing composition may further comprise purified water, wherein the content of the biodegradable scrub particles may be 0.5 to 20 parts by weight relative to 100 parts by weight of the purified water.

According to another embodiment of the present disclosure, the skin cleansing composition may further comprise purified water, wherein the content of the surfactant may be 20 to 60 parts by weight, and the content of the polyol may be 0.5 to 25 parts by weight, relative to 100 parts by weight of the purified water.

According to another embodiment of the present disclosure, the surfactant may comprise at least one selected from the group consisting of cocamidopropyl betaine, potassium cocoyl glycinate, sodium olivate, stearic acid, myristic acid, lauric acid, glyceryl stearate, sodium lauryl sulfate, sodium laureth sulfate, sodium coco sulfate, sodium cocoyl isethionate, sodium lauroyl methyl isethionate, sodium lauroyl glutamate, sodium lauroyl aspartate, polysorbate 60, polysorbate 80, lauryl hydroxysultaine, sorbeth-30 tetraoleate, sucrose laurate, polyglyceryl-10 oleate, polyglyceryl-10 dioleate, polyglyceryl-2 sesquicaprylate, and laureth-4.

According to another embodiment of the present disclosure, the polyol may comprise at least one selected from the group consisting of glycerin, 1,3-butylene glycol, dipropylene glycol, propylene glycol, polyethylene glycol, and 1,2-hexanediol.

According to another embodiment of the present disclosure, the skin cleansing composition may further comprise at least one selected from the group consisting of a moisturizer, a softener, a thickener, a preservative, a neutralizer, an acidity regulator, and a fragrance.

According to another embodiment of the present disclosure, the skin cleansing composition may be for cleansing the face, body, or scalp.

### Advantageous Effects of Invention

The biodegradable scrub particles according to the present disclosure comprise a polyhydroxyalkanoate (PHA) while the particle size thereof is controlled to a specific range; thus, when the skin is cleansed with a skin cleansing composition comprising the particles, the skin cleansing power is excellent for removing skin impurities and wastes (e.g., sebum, dead skin cells, etc.) or residual cosmetics, thereby making the skin texture smooth and maintaining an ideal skin condition.

In addition, the biodegradable scrub particles comprising a polyhydroxyalkanoate (PHA) have a smooth and uniform surface, so that they can be rolled smoothly without damaging the skin. As a result, when the skin is cleansed with a skin cleansing composition comprising the biodegradable scrub particles, it causes little irritation to the skin and imparts softness to the skin after cleansing.

In addition, the polyhydroxyalkanoate (PHA) contained in the biodegradable scrub particles has excellent biodegradability in soil and/or the ocean; thus, the biodegradable scrub particles according to the present disclosure can be environmentally friendly without causing microplastic problems.

Accordingly, a skin cleansing composition comprising the biodegradable scrub particles according to the present disclosure can be effectively applied to cleansing products (cosmetic products) for cleansing the face, body, or scalp (hair), and it can also effectively act for environmental conservation.

### Best Mode for Carrying out the Invention

Hereinafter, the present disclosure will be described in detail. The present disclosure is not limited to those given below, but it may be modified into various forms as long as the gist of the disclosure is not changed.

In the present specification, the term "comprising" is intended to specify a particular characteristic, region, step, process, element, and/or component. It does not exclude the presence or addition of any other characteristic, region, step, process, element and/or component, unless specifically stated to the contrary.

All numbers and expressions related to the quantities of components, reaction conditions, and the like used herein are to be understood as being modified by the term "about" unless otherwise indicated.

### Biodegradable scrub particles

The biodegradable scrub particles (hereinafter referred to as "scrub particles") according to the present disclosure comprise a polyhydroxyalkanoate (hereinafter referred to as "PHA") while the particle size thereof is controlled to a specific range. For example, the scrub particles may be composed of PHA particles, which are described in detail as follows.

The PHA particles, which are scrub particles, have a particle size (average particle diameter or median particle size; the particle size when the cumulative percentage reaches 50%, D₅₀) of 10 to 1,000 µm. As the size (D₅₀) of the PHA particles is within the above range, rolling on the skin is performed well without damaging the skin, and a skin cleansing composition containing the same can have excellent skin cleansing power with little irritation to the skin. Specifically, the size (D₅₀) of the PHA particles may be 10 to 950 µm, 10 to 900 µm, 10 to 850 µm, 10 to 800 µm, 20 to 950 µm, 30 to 900 µm, 40 to 850 µm, 50 to 800 µm, 60 to 750 µm, 70 to 700 µm, 80 to 650 µm, 85 to 650 µm, 90 to 600 µm, 95 to 550 µm, 100 to 500 µm, 150 to 450 µm, or 200 to 400 µm, but it is not limited thereto.

For example, when the skin cleansing composition that comprises the PHA particles as scrub particles is applied to a face cleansing product, the size (D₅₀) of the PHA particles may be 10 to 400 µm, 15 to 380 µm, 20 to 360 µm, 25 to 350 µm, 30 to 340 µm, 35 to 330 µm, 40 to 320 µm, 45 to 310 µm, or 50 to 300 µm, but it is not limited thereto. In addition, when the skin cleansing composition that comprises the PHA particles as scrub particles is applied to a scalp (hair) and/or body cleansing product, the size (D₅₀) of the PHA particles may be 10 to 900 µm, 30 to 880 µm, 50 to 860 µm, 70 to 840 µm, 90 to 820 µm, 100 to 800 µm, 150 to 750 µm, 180 to 700 µm, 200 to 600 µm, or 200 to 550 µm, but it is not limited thereto.

The PHA particles may be obtained by particleizing a PHA using a conventional method; and then feeding and sorting the particleized PHA into a circular vibration sorter.

The PHA, which is a natural polyester polymer that accumulates within microbial cells, can be completely decomposed into carbon dioxide, water, organic waste, and the like in soil and/or the ocean; thus, it can have excellent biodegradability without generating microplastics. Accordingly, the scrub particles according to the present disclosure can be excellent in biodegradability and environmental friendliness as they comprise such a PHA.

The PHA may be obtained by cell disruption using a mechanical method or a physical method, or it may be obtained by cell disruption using a non-mechanical method or a chemical method. Specifically, the PHA may be a copolymer obtained through an enzyme-catalyzed polymerization process of one or more monomers within living microbial cells.

The PHA may comprise a repeat unit derived from at least one (e.g., two or more) monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH). Specifically, the PHA may comprise a repeat unit derived from at least one (e.g., two or more) monomer selected from the group consisting of 3-hydroxybutyrate (3-HB), 3-hydroxypropionate (3-HP), 3-hydroxyvalerate (3-HV), 3-hydroxyhexanoate (3-HH), 4-hydroxybutyrate (4-HB), 4-hydroxyvalerate (4-HV), 5-hydroxyvalerate (5-HV), and 6-hydroxyhexanoate (6-HH). In such an event, the repeat unit(s) derived from the at least one monomer may be randomly distributed.

More specifically, the PHA comprises a repeat unit derived from 4-hydroxybutyrate (4-HB) while it may further comprise 1, 2, 3, 4, 5, 6, or more repeat units derived from monomer(s) different from the 4-hydroxybutyrate (4-HB). As the PHA comprises a repeat unit derived from 4-hydroxybutyrate (4-HB), the properties of the scrub particles can be optimized. As a result, it is possible to provide a skin cleansing composition that can be used for various purposes.

The PHA may be classified into a crystalline PHA (cPHA), a semi-crystalline PHA (scPHA), or an amorphous PHA (aPHA) depending on the types of monomers and the content of repeat units derived from the monomers. For example, the PHA may be classified into cPHA, scPHA, or aPHA as its crystallinity is controlled depending on the content of a repeat unit derived from 4-hydroxybutyrate (4-HB).

Specifically, the PHA may comprise a repeat unit derived from 3-hydroxybutyrate (3-HB) (hereinafter referred to as "3-HB repeat unit") and a repeat unit derived from 4-hydroxybutyrate (4-HB) (hereinafter referred to as "4-HB repeat unit"). That is, the PHA may be a poly(3-hydroxybutyrate-co-4-hydroxybutyrate; P3HB-co-4HB) copolymer that comprises the 3-HB repeat unit and the 4-HB repeat unit. As the PHA comprises the 3-HB repeat unit and the 4-HB repeat unit, the mechanical strength, thermal resistance, oil resistance, and the like of the scrub particles are secured, and even when the skin cleansing composition comprising the scrub particles is used for various purposes (e.g., face, body, or scalp, etc.), the skin cleansing power can be secured.

It may be important to control the content of the 4-HB repeat unit contained in the PHA in order to provide scrub particles and a skin cleansing composition having the desired properties in the present disclosure. Specifically, the content of the 4-HB repeat unit contained in the PHA may be 0.1 to 50% by weight based on the total weight of the PHA. More specifically, the content of the 4-HB repeat unit may be 0.1 to 45% by weight, 0.2 to 43% by weight, 0.3 to 40% by weight, 0.3 to 38% by weight, 0.3 to 35% by weight, 0.4 to 32% by weight, 0.4 to 30% by weight, 0.5 to 28% by weight, 0.5 to 25% by weight, 0.6 to 20% by weight, 0.6 to 18% by weight, 0.7 to 16% by weight, 0.8 to 15% by weight, 1 to 15% by weight, 1 to 13% by weight, 1 to 10% by weight, 1.5 to 10% by weight, 1.8 to 10% by weight, 2 to 10% by weight, 2.5 to 9.5% by weight, 3 to 9% by weight, 3.5 to 8% by weight, 4 to 7% by weight, or 5 to 6.5% by weight, based on the total weight of the PHA, but it is not limited thereto. As the content of the 4-HB repeat unit is within the above range, the biodegradability of the scrub particles can be further increased, while the formulation stability and mechanical strength of the scrub particles can be secured at the desired levels.

The weight average molecular weight (Mw) of the PHA may be 100,000 to 800,000 g/mole, 130,000 to 780,000 g/mole, 150,000 to 750,000 g/mole, 200,000 to 700,000 g/mole, 250,000 to 650,000 g/mole, 280,000 to 600,000 g/mole, 300,000 to 550,000 g/mole, or 320,000 to 500,000 g/mole, but it is not limited thereto.

### Skin cleansing composition

The skin cleansing composition according to the present disclosure comprises scrub particles that comprise a PHA while the particle size thereof is controlled to a specific range. Specifically, the skin cleansing composition according to the present disclosure comprises biodegradable scrub particles comprising a PHA; a surfactant; and a polyol, and optionally further comprises purified water and/or an additive, which are described in detail as follows.

The biodegradable scrub particles contained in the skin cleansing composition according to the present disclosure comprise the PHA described above. Specifically, the biodegradable scrub particles have substantially the same constitution and characteristics as those of the scrub particles described above. As the skin cleansing composition according to the present disclosure comprises such biodegradable scrub particles, it can have excellent biodegradability, skin cleansing power, and formulation stability, along with little irritation to the skin.

The content of the biodegradable scrub particles is not particularly limited, but it may be 0.5 to 30% by weight based on the total weight of the skin cleansing composition. Specifically, the content of the biodegradable scrub particles may be 0.5 to 25% by weight, 0.5 to 23% by weight, 0.5 to 20% by weight, 0.6 to 18% by weight, 0.6 to 15% by weight, 0.7 to 13% by weight, 0.8 to 12% by weight, 1 to 11% by weight, 2 to 10% by weight, or 3 to 10% by weight, based on the total weight of the skin cleansing composition, but it is not limited thereto.

Here, when the skin cleansing composition further comprises purified water, the content of the biodegradable scrub particles may be 0.5 to 20 parts by weight relative to 100 parts by weight of the purified water. Specifically, the content of the biodegradable scrub particles may be 1 to 18 parts by weight, 1.5 to 17 parts by weight, 2 to 16 parts by weight, 2.5 to 15 parts by weight, 3 to 14 parts by weight, 3.5 to 13 parts by weight, 4 to 12 parts by weight, 4.5 to 11 parts by weight, or 5 to 10 parts by weight, relative to 100 parts by weight of the purified water, but it is not limited thereto.

As the content of the biodegradable scrub particles is within the above range, a skin cleansing composition having excellent skin cleansing power, little irritation to the skin, and secured formulation stability can be provided.

The surfactant contained in the skin cleansing composition according to the present disclosure is an ingredient that improves the skin cleansing power of the skin cleansing composition while it allows the skin cleansing composition to be readily rinsed with water.

The surfactant is not particular limited, but it may comprise at least one selected from the group consisting of cocamidopropyl betaine, potassium cocoyl glycinate, sodium olivate, stearic acid, myristic acid, lauric acid, glyceryl stearate, sodium lauryl sulfate, sodium laureth sulfate, sodium coco sulfate, sodium cocoyl isethionate, sodium lauroyl methyl isethionate, sodium lauroyl glutamate, sodium lauroyl aspartate, polysorbate 60, polysorbate 80, lauryl hydroxysultaine, sorbeth-30 tetraoleate, sucrose laurate, polyglyceryl-10 oleate, polyglyceryl-10 dioleate, polyglyceryl-2 sesquicaprylate, and laureth-4. Specifically, the surfactant may comprise two or more (specifically, three or more, four or more, or five or more) selected from the group consisting of cocamidopropyl betaine, potassium cocoyl glycinate, sodium olivate, stearic acid, myristic acid, lauric acid, glyceryl stearate, sodium lauryl sulfate, lauryl hydroxysultaine, and laureth-4.

The content of the surfactant is not particularly limited, but it may be 10 to 40% by weight based on the total weight of the skin cleansing composition. Specifically, the content of the surfactant may be 12 to 40% by weight, 13 to 38% by weight, 14 to 36% by weight, 15 to 35% by weight, 16 to 33% by weight, 17 to 32% by weight, 18 to 31% by weight, 19 to 30% by weight, 20 to 28% by weight, or 22 to 27% by weight, based on the total weight of the skin cleansing composition, but it is not limited thereto.

In addition, when the skin cleansing composition further comprises purified water, the content of the surfactant may be 20 to 60 parts by weight relative to 100 parts by weight of the purified water. Specifically, the content of the surfactant may be 20 to 58 parts by weight, 22 to 55 parts by weight, 25 to 53 parts by weight, 28 to 50 parts by weight, 30 to 48 parts by weight, 32 to 45 parts by weight, 33 to 43 parts by weight, 34 to 42 parts by weight, or 35 to 40 parts by weight, relative to 100 parts by weight of the purified water, but it is not limited thereto.

As the content of the surfactant is within the above range, a skin cleansing composition can be provided in which skin cleansing power and rinsing stability are secured, along with minimized irritation to the skin.

The polyol contained in the skin cleansing composition according to the present disclosure is an ingredient that secures the formulation stability of the skin cleansing composition while it increases skin moisturizing power.

The polyol is not particularly limited, but it may comprise at least one selected from the group consisting of glycerin, 1,3-butylene glycol, dipropylene glycol, propylene glycol, polyethylene glycol, and 1,2-hexanediol. Specifically, the polyol may comprise glycerin, which is economical and has excellent moisturizing power.

The content of the polyol is not particularly limited, but it may be 1 to 15% by weight based on the total weight of the skin cleansing composition. Specifically, the content of the polyol may be 1.5 to 15% by weight, 2 to 14.5% by weight, 3 to 14% by weight, 4 to 13.5% by weight, 5 to 13% by weight, 6 to 12.5% by weight, 7 to 12% by weight, 7.5 to 11.5% by weight, 8 to 11% by weight, or 8.5 to 11% by weight, based on the total weight of the skin cleansing composition, but it is not limited thereto.

In addition, when the skin cleansing composition further comprises purified water, the content of the polyol may be 0.5 to 25 parts by weight relative to 100 parts by weight of the purified water. Specifically, the content of the polyol may be 0.8 to 24 parts by weight, 1 to 22 parts by weight, 1.5 to 20 parts by weight, 1.8 to 18 parts by weight, 2 to 17 parts by weight, 2.2 to 16 parts by weight, 2.4 to 15 parts by weight, 2.6 to 14 parts by weight, or 2.8 to 13.5 parts by weight, relative to 100 parts by weight of the purified water, but it is not limited thereto.

As the content of the polyol is within the above range, a skin cleansing composition with secured skin moisturizing power and formulation stability can be provided.

The skin cleansing composition according to the present disclosure may further comprise purified water to control viscosity and secure formulation stability. The purified water is not particularly limited as long as it is commonly known purified water (e.g., distilled water, deionized water, pure water, or the like).

The skin cleansing composition according to the present disclosure may further comprise at least one additive selected from the group consisting of a moisturizer, a softener, a thickener, a preservative, a neutralizer, an acidity regulator, and a fragrance, as needed.

The moisturizer further contained in the skin cleansing composition according to the present disclosure is an ingredient capable of supplying moisture to the skin.

The softener further contained in the skin cleansing composition according to the present disclosure is an ingredient capable of imparting softness to the skin while soothing the skin. The softener is not particularly limited, but it may comprise at least one selected from the group consisting of natural vegetable oils, ester-based oils, hydrocarbon-based oils, higher alcohols, and silicone oils.

The thickener further contained in the skin cleansing composition according to the present disclosure is an ingredient that increases the viscosity of the skin cleansing composition. The thickener is not particularly limited, but it may comprise at least one selected from the group consisting of polyquaternium-10, carbomer, sodium chloride, methyl cellulose, carboxymethyl cellulose, carboxymethyl hydroxyguanine, hydroxymethyl cellulose, hydroxyethyl cellulose, sodium acryloyldimethyltaurate/VP crosspolymer, ammonium acryloyldimethyltaurate/VP copolymer, hydroxyethyl acrylate/sodium acryloyldimethyl taurate copolymer, polyacrylate crosspolymer-6, stearic acid, carrageenan, xanthan gum, gellan gum, cellulose gum, and bentonite.

The preservative further contained in the skin cleansing composition according to the present disclosure is an ingredient that increases the storage stability of the skin cleansing composition. The preservative is not particularly limited, but it may comprise at least one selected from the group consisting of phenoxyethanol, ethylhexylglycerin, caprylyl glycol, chlorphenesin, and sodium benzoate.

The neutralizer further contained in the skin cleansing composition according to the present disclosure is an ingredient that interacts with the preservative contained in the skin cleansing composition to increase the safety of the skin cleansing composition during its use. The neutralizer is not particularly limited, but it may comprise at least one selected from the group consisting of potassium hydroxide, sodium hydroxide, calcium hydroxide, triethanolamine, trolamine, and aminomethyl propanol.

The acidity regulator further contained in the skin cleansing composition according to the present disclosure is an ingredient that regulates the pH of the skin cleansing composition. The acidity regulator is not particularly limited, but it may comprise at least one selected from the group consisting of citric acid, acetic acid, propionic acid, oxalic acid, glycolic acid, malonic acid, lactic acid, succinic acid, tartaric acid, aspartic acid, maleic acid, glutaric acid, glutamic acid, gluconic acid, sorbic acid, benzoic acid, ascorbic acid, and salicylic acid.

The fragrance further contained in the skin cleansing composition according to the present disclosure is an ingredient that imparts fragrance to the skin cleansing composition.

The content of these additives can be appropriately adjusted within a range that does not impair the properties required for the skin cleansing composition.

As the skin cleansing composition according to the present disclosure comprises the ingredients described above, it can secure formulation stability while having excellent biodegradability and skin cleansing power. In particular, as the skin cleansing composition according to the present disclosure comprises the biodegradable scrub particles described above, it is biodegradable to be environmentally friendly while it causes little irritation to the skin and imparts softness to the skin after cleansing.

The skin cleansing composition according to the present disclosure can be used for various purposes. For example, the skin cleansing composition according to the present disclosure can be advantageously applied to a cleansing product for cleansing the face, body, or scalp.

### Mode for the Invention

Hereinafter, the present disclosure will be described in detail with reference to Examples, but the scope of the present disclosure is not limited to the Examples.

### [Example 1] Preparation of biodegradable scrub particles

A polyhydroxyalkanoate comprising a 3-HB repeat unit and a 4-HB repeat unit (P3HB-co-4HB having a content of 4-HB repeat units of 6% by weight; CJ CheilJedang) was particleized using an atomizer pulverizing device to prepare a powdered polyhydroxyalkanoate. Next, the prepared polyhydroxyalkanoate was fed to a circular vibration sorter and subjected to a sorting procedure to produce polyhydroxyalkanoate particles with a D₅₀ of 250 to 350 µm as scrub particles.

### [Example 2] Preparation of biodegradable scrub particles

Polyhydroxyalkanoate particles having a D₅₀ of 250 to 350 µm were prepared as scrub particles through the same procedure as in Example 1, except that a P3HB-co-4HB having a content of 12% by weight of 4-HB repeat units was used.

### <Skin cleansing composition - preparation of a body wash>

### [Preparation Example 1]

Step (1): An aqueous phase (Raw Materials 1 and 2) was charged to a beaker at room temperature. While it was mixed using an Agi mixer, surfactants (Raw Materials 3 to 5) were slowly added thereto over 5 minutes. Upon completion of the addition, they were mixed for 10 minutes to obtain a homogenized Dispersion 1.

Step (2): A thickener (Raw Material 6) and a neutralizer (Raw Material 7) were slowly added to Dispersion 1 of step (1) at room temperature over 5 minutes, and they were mixed to obtain a homogenized Dispersion 2.

Step (3): Preservatives (Raw Materials 8 and 9) were slowly added to Dispersion 2 of step (2) at room temperature over 5 minutes, and they were mixed to obtain a homogenized Dispersion 3.

Step (4): The scrub particles (Raw Material 10) of Example 1 were slowly added to Dispersion 3 of step (3) at room temperature over 5 minutes, and they were stirred to prepare a transparent gel-like body wash.

### [Preparation Example 2]

A body wash was prepared through the same procedure as in Preparation Example 1, except that the scrub particles (Raw Material 10) of Example 2 were used while the composition (unit: parts by weight) was as shown in Table 1 below.

### [Comparative Preparation Example 1]

A body wash was prepared through the same procedure as in Preparation Example 1, except that step (4) of adding the scrub particles of Example 1 with stirring was omitted while the composition (unit: parts by weight) was as shown in Table 1 below.

**[Table 1]**

| Raw material | Component | Prep. Ex. 1 | Prep. Ex. 2 | C. Prep. Ex. 1 |
|---|---|---|---|---|
| 1 | Water (purified water) | to 100 | to 100 | to 100 |
| 2 | Glycerin (polyol) | 10 | 10 | 10 |
| 3 | Cocamidopropyl betaine (surfactant) | 20 | 20 | 20 |
| 4 | Potassium cocoyl glycinate (surfactant) | 12 | 12 | 12 |
| 5 | Sodium olivate (surfactant) | 5 | 5 | 5 |
| 6 | Carbomer 2% solution (thickener) | 6 | 6 | 6 |
| 7 | Potassium hydroxide 10% solution (neutralizer) | 0.5 | 0.5 | 0.5 |
| 8 | Phenoxyethanol (preservative) | 0.45 | 0.45 | 0.45 |
| 9 | Ethylhexylglycerin (preservative) | 0.05 | 0.05 | 0.05 |
| 10 | PHA with a 4HB repeat unit content of 6% by weight (scrub particles) | 10 | - | - |
| | PHA with a 4HB repeat unit content of 12% by weight (scrub particles) | - | 10 | - |

### [Test Example 1] Evaluation of usability satisfaction

The usability satisfaction of the body washes prepared in Preparation Examples 1 and 2 and Comparative Preparation Example 1 was evaluated. The results are shown in Table 2 below. Specifically, 5 g of each body wash was applied to the arms of ten women in their 30s and 40s and ten men in their 30s and 40s and rolled 10 times, respectively. Subsequently, it was wetted with water, rolled again 10 times, and washed off. The usability was scored for each evaluation item, and the average score was calculated.

### * Evaluation criteria

Very good: 5
Good: 4
Normal: 3
Poor: 2
Very poor: 1

**[Table 2]**

| Evaluation item | Prep. Ex. 1 | Prep. Ex. 2 | C. Prep. Ex. 1 |
|---|---|---|---|
| Rolling feeling | 4.3 | 3.9 | 3.5 |
| Cleansing power | 4.5 | 3.9 | 3.5 |
| Finishing touch | 4.4 | 4.0 | 3.0 |
| Usability satisfaction | 95% | 85% | 62% |

Referring to Table 2 above, the body washes of Preparation Examples 1 and 2, comprising the biodegradable scrub particles according to the present disclosure, had good overall usability satisfaction. In particular, the body wash of Preparation Example 1, in which scrub particles having a content of 10% by weight or less of 4-HB repeat units contained in the polyhydroxyalkanoate were used, was good in rolling feeling, excellent in cleansing power, and not dry, along with finishing touch without residual feeling.

Meanwhile, the body wash of Comparative Preparation Example 1, in which biodegradable scrub particles were not used, had poor overall usability satisfaction.

### <Skin cleansing composition - preparation of a cleansing foam>

### [Preparation Example 3]

Step (1): Surfactants (Raw Materials 3 to 7) were charged to a beaker and stirred using an Agi mixer at 75°C to obtain a mixed solution.

Step (2): A polyol (Raw Material 2) and the mixed solution of step (1) were added to purified water (Raw Material 1) heated to 75°C, and they were stirred at 3,500 rpm for 5 minutes using a homomixer (Premix, Japan) to obtain an emulsion.

Step (3): The emulsion of step (2) was cooled to 50°C, and a neutralizer (Raw Material 8) was then slowly added thereto. They were stirred at 3,500 rpm for 5 minutes using a homomixer (Premix, Japan) to adjust the viscosity of the emulsion.

Step (4): The scrub particles (Raw Material 9) of Example 1 were added to the emulsion whose viscosity had been adjusted in step (3) at 45°C, and they were stirred to prepare a cleansing foam.

### [Preparation Example 4]

A cleansing foam was prepared through the same procedure as in Preparation Example 3, except that the scrub particles of Example 2 were used while the composition (unit: parts by weight) was as shown in Table 3 below.

### [Comparative Preparation Example 2]

A cleansing foam was prepared through the same procedure as in Preparation Example 3, except that step (4) of adding the scrub particles of Example 1 with stirring was omitted while the composition (unit: parts by weight) was as shown in Table 3 below.

**[Table 3]**

| Raw material | Item | Prep. Ex. 3 | Prep. Ex. 4 | C. Prep. Ex. 2 |
|---|---|---|---|---|
| 1 | Water (purified water) | to 100 | to 100 | to 100 |
| 2 | Glycerin (polyol) | 13 | 13 | 13 |
| 3 | Stearic acid (surfactant) | 18 | 18 | 18 |
| 4 | Myristic acid (surfactant) | 7 | 7 | 7 |
| 5 | Lauric acid (surfactant) | 5 | 5 | 5 |
| 6 | Glyceryl stearate (surfactant) | 2 | 2 | 2 |
| 7 | Lauryl hydroxysultaine (surfactant) | 3 | 3 | 3 |
| 8 | Potassium hydroxide 10% solution (neutralizer) | 0.1 | 0.1 | 0.1 |
| 9 | PHA with a 4HB repeat unit content of 6% by weight (scrub particles) | 5 | - | - |
| | PHA with a 4HB repeat unit content of 12% by weight (scrub particles) | - | 5 | - |

### [Test Example 2] Evaluation of the cleansing effect

The cleansing effect of the cleansing foams prepared in Preparation Examples 3 and 4 and Comparative Preparation Example 2 was evaluated. The results are shown in Table 4 below. Specifically, various makeup products were applied to the backs of the hands of ten women in their 30s and 40s and ten men in their 30s and 40s, respectively. Next, 2 g of each cleansing foam was applied to the back of the hand, which was wetted with water, rolled 10 times, and washed off. The remaining amount of makeup product was evaluated, and the average was calculated.

### * Evaluation criteria

Very good: 0 to less than 5% of remaining makeup product after cleansing
Good: 5 to less than 25% of remaining makeup product after cleansing
Poor: 25 to less than 50% of remaining makeup product after cleansing
Very poor: 50 to 100% of remaining makeup product after cleansing

**[Table 4]**

| Evaluation item | Prep. Ex. 3 | Prep. Ex. 4 | C. Prep. Ex. 2 |
|---|---|---|---|
| Cleansing effect | Very good | Good | Poor |

Referring to Table 4 above, the cleansing foams of Preparation Examples 3 and 4, comprising the biodegradable scrub particles according to the present disclosure, had a good cleansing effect (cleansing power). In contrast, the cleansing foam of Comparative Preparation Example 2, in which biodegradable scrub particles were not used, had a poor cleansing effect.

### [Test Example 3] Evaluation of the removal effect of dead skin cells

The removal effect of dead skin cells of the cleansing foams prepared in Preparation Examples 3 and 4 and Comparative Preparation Example 2 was evaluated. The results are shown in Table 5 below. Specifically, 2 g of each cleansing foam was applied to the backs of the hands of ten women in their 30s and 40s and ten men in their 30s and 40s, respectively, which was wetted with water, rolled 10 times to create foam, and then washed off. Next, the degree of dead skin cells removed from the back of the hand was evaluated using a callus tape (BOMTECH, CORNEUM TYPE) device, and the average was calculated.

### * Evaluation criteria

1: 95% or more of dead skin cells were removed relative to the state before use
2: 60% to less than 80% of dead skin cells were removed relative to the state before use
3: 40% to less than 60% of dead skin cells were removed relative to the state before use
4: Less than 40% of dead skin cells were removed relative to the state before use

**[Table 5]**

| Evaluation item | Prep. Ex. 3 | Prep. Ex. 4 | C. Prep. Ex. 2 |
|---|---|---|---|
| Removal effect of dead skin cells | 1.5 | 2 | 4 |

Referring to Table 5 above, the cleansing foams of Preparation Examples 3 and 4, comprising the biodegradable scrub particles according to the present disclosure, had a good effect on removing dead skin cells. In contrast, the cleansing foam of Comparative Preparation Example 2, in which biodegradable scrub particles were not used, had a very poor effect on removing dead skin cells.

### <Skin cleansing composition - preparation of a shampoo>

### [Preparation Example 5]

Step (1): Purified water (Raw Material 1), a polyol (Raw Material 2), and a thickener (Raw Material 3) were charged to a beaker. They were stirred using an Agi mixer while being heated at 45°C to obtain a mixed solution.

Step (2): While surfactants (Raw Materials 4 to 6) and a softener (Raw Material 7) were slowly added to the mixed solution of step (1) at 45°C, they were stirred at 500 rpm for 5 minutes using an Agi mixer to obtain Dispersion 1.

Step (3): While the temperature was maintained at 45°C, preservatives (Raw Materials 8 and 9) were slowly added to Dispersion 1 of step (2), and they were stirred at 500 rpm for 5 minutes using an Agi mixer to obtain Dispersion 2.

Step (4): While the temperature was maintained at 45°C, an acidity regulator (Raw Material 10) was added to Dispersion 2 of step (3) to adjust the pH. Then, while a thickener (Raw Material 11) was slowly added thereto, they were stirred at 700 rpm for 5 minutes using an Agi mixer to obtain Dispersion 3.

Step (5): While the temperature was maintained at 45°C, the scrub particles (Raw Material 12) of Example 1 were added to Dispersion 3 of step (4), and they were stirred at 700 rpm for 5 minutes using an Agi mixer to prepare a shampoo.

### [Preparation Example 6]

A shampoo was prepared through the same procedure as in Preparation Example 5, except that the scrub particles of Example 2 were used while the composition (unit: parts by weight) was as shown in Table 6 below.

### [Comparative Preparation Example 3]

A shampoo was prepared through the same procedure as in Preparation Example 5, except that step (5) of adding the scrub particles of Example 1 with stirring was omitted while the composition (unit: parts by weight) was as shown in Table 6 below.

**[Table 6]**

| Raw material | Component | Prep. Ex. 5 | Prep. Ex. 6 | C. Prep. Ex. 3 |
|---|---|---|---|---|
| 1 | Water (purified water) | to 100 | to 100 | to 100 |
| 2 | Glycerin (polyol) | 3 | 3 | 3 |
| 3 | Polyquaternium 10 (thickener) | 0.4 | 0.4 | 0.4 |
| 4 | Cocamidopropyl betaine (surfactant) | 12 | 12 | 12 |
| 5 | Sodium laureth sulfate (surfactant) | 23 | 23 | 23 |
| 6 | Laureth 4 (surfactant) | 4 | 4 | 4 |
| 7 | Dimethicone (softener) | 3 | 3 | 3 |
| 8 | Phenoxyethanol (preservative) | 0.45 | 0.45 | 0.45 |
| 9 | Ethylhexylglycerin (preservative) | 0.05 | 0.05 | 0.05 |
| 10 | Citric acid (acidity regulator) | 1 | 1 | 1 |
| 11 | Sodium chloride (thickener) | 1 | 1 | 1 |
| 12 | PHA with a 4HB repeat unit content of 6% by weight (scrub particles) | 5 | - | - |
| | PHA with a 4HB repeat unit content of 12% by weight (scrub particles) | - | 5 | - |

### [Test Example 4] Evaluation of scalp cleansing effect

The scalp cleansing effect of the shampoos prepared in Preparation Examples 5 and 6 and Comparative Preparation Example 3 was evaluated. The results are shown in Tables 7 and 8 below. Specifically, ten women in their 30s and 40s and ten men in their 30s and 40s were each asked to use 5 ml of each shampoo per use on their scalps. Then, the feeling of use was scored for each evaluation item, and the average score was calculated. Meanwhile, while the usability evaluation as described above was conducted, a questionnaire on irritation was administered.

### * Evaluation criteria

Very good: 5
Good: 4
Normal: 3
Poor: 2
Very poor: 1
High: high irritation
Medium: medium irritation
Low: no irritation

**[Table 7]**

| Evaluation item | Prep. Ex. 5 | Prep. Ex. 6 | C. Prep. Ex. 3 |
|---|---|---|---|
| Scalp application feeling | 4.2 | 4.0 | 3.5 |
| Scalp massage feeling | 4.5 | 4.1 | 3.5 |
| Scalp refreshment feeling | 4.7 | 4.2 | 2.5 |
| Usability satisfaction | 96% | 85% | 60% |

**[Table 8]**

| Evaluation item | Prep. Ex. 5 | Prep. Ex. 6 | C. Prep. Ex. 3 |
|---|---|---|---|
| Irritation | Medium | Medium | Low |

Referring to Table 7 above, the shampoos of Preparation Examples 5 and 6, comprising the biodegradable scrub particles according to the present disclosure, had a good scalp massage feeling and refreshment feeling, resulting in high usability satisfaction. In contrast, the shampoo of Comparative Preparation Example 3, in which biodegradable scrub particles were not used, had a poor scalp refreshment feeling, resulting in low usability satisfaction. Meanwhile, referring to Table 8 above, the shampoos of Preparation Examples 5 and 6, comprising the biodegradable scrub particles, received an average "medium" rating in the irritation evaluation, indicating no stinging or discomfort, despite the use of biodegradable scrub particles. This supports that the biodegradable scrub particles according to the present disclosure have little irritation to the skin.

### [Test Example 5] Evaluation of formulation stability

The formulation stability of the skin cleansing compositions prepared in Preparation Examples 1 to 6 and Comparative Preparation Examples 1 to 3 was evaluated. The results are shown in Table 9 below. Specifically, the skin cleansing compositions were stored at each temperature condition of 4°C, 25°C, 45°C, and 50°C for 1 week, 2 weeks, and 1 month. It was checked whether precipitation and separation, discoloration, and odor change occurred.

### * Evaluation criteria

∘: No precipitation, separation, discoloration, or odor change occurred.
×: Precipitation, separation, discoloration, or odor change occurred.

**[Table 9]**

| Storage Temp. | Storage period | Prep. Ex. 1 | Prep. Ex. 2 | Prep. Ex. 3 | Prep. Ex. 4 | Prep. Ex. 5 | Prep. Ex. 6 | C. Prep. Ex. 1 | C. Prep. Ex. 2 | C. Prep. Ex. 3 |
|---|---|---|---|---|---|---|---|---|---|---|
| 4°C | 1 week | ○ | ○ | ○ | ○ | ○ | ○ | ○ | ○ | ○ |
| | 2 weeks | ○ | ○ | ○ | ○ | ○ | ○ | ○ | ○ | ○ |
| | 1 month | ○ | ○ | ○ | ○ | ○ | ○ | ○ | ○ | ○ |
| 25°C | 1 week | ○ | ○ | ○ | ○ | ○ | ○ | ○ | ○ | ○ |
| | 2 weeks | ○ | ○ | ○ | ○ | ○ | ○ | ○ | ○ | ○ |
| | 1 month | ○ | ○ | ○ | ○ | ○ | ○ | ○ | ○ | ○ |
| 45°C | 1 week | ○ | ○ | ○ | ○ | ○ | ○ | ○ | ○ | ○ |
| | 2 weeks | ○ | ○ | ○ | ○ | ○ | ○ | ○ | ○ | ○ |
| | 1 month | ○ | ○ | ○ | ○ | ○ | ○ | ○ | ○ | ○ |
| 50°C | 1 week | ○ | ○ | ○ | ○ | ○ | ○ | ○ | ○ | ○ |
| | 2 weeks | ○ | ○ | ○ | ○ | ○ | ○ | ○ | ○ | ○ |
| | 1 month | ○ | × | ○ | × | ○ | × | ○ | ○ | ○ |

Referring to Table 9 above, the skin cleansing compositions of Preparation Examples 1 to 6 according to the present disclosure had excellent overall formulation stability even when they comprised biodegradable scrub particles. In particular, when the scrub particles having a content of 10% by weight or less of 4-HB repeat units contained in the polyhydroxyalkanoate were used, the formulation stability was excellent even at a temperature of 45°C or higher.

## Claims

1. Biodegradable scrub particles, which comprise a polyhydroxyalkanoate (PHA) comprising a repeat unit derived from 4-hydroxybutyrate (4-HB) in an amount of 0.1 to 50% by weight based on the total weight of the polyhydroxyalkanoate (PHA), wherein the particle size (D₅₀) of the biodegradable scrub particles is 10 to 1,000 µm.

2. The biodegradable scrub particles of claim 1, wherein the polyhydroxyalkanoate (PHA) further comprises a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

3. The biodegradable scrub particles of claim 1, wherein the polyhydroxyalkanoate (PHA) further comprises a repeat unit derived from 3-hydroxybutyrate (3-HB).

4. The biodegradable scrub particles of claim 1, wherein the particle size (D₅₀) of the biodegradable scrub particles is 10 to 800 µm.

5. The biodegradable scrub particles of claim 1, wherein the content of the repeat unit derived from 4-hydroxybutyrate (4-HB) is 1 to 10% by weight based on the total weight of the polyhydroxyalkanoate (PHA).

6. A skin cleansing composition, which comprises the biodegradable scrub particles of claim 1; a surfactant; and a polyol.

7. The skin cleansing composition of claim 6, wherein the content of the biodegradable scrub particles is 0.5 to 30% by weight based on the total weight of the skin cleansing composition.

8. The skin cleansing composition of claim 6, wherein the content of the surfactant is 10 to 40% by weight, and the content of the polyol is 1 to 15% by weight, based on the total weight of the skin cleansing composition.

9. The skin cleansing composition of claim 6, wherein the skin cleansing composition further comprises purified water, and
the content of the biodegradable scrub particle is 0.5 to 20 parts by weight relative to 100 parts by weight of the purified water.

10. The skin cleansing composition of claim 6, wherein the skin cleansing composition further comprises purified water, and
the content of the surfactant is 20 to 60 parts by weight, and the content of the polyol is 0.5 to 25 parts by weight, relative to 100 parts by weight of the purified water.

11. The skin cleansing composition of claim 6, wherein the surfactant comprises at least one selected from the group consisting of cocamidopropyl betaine, potassium cocoyl glycinate, sodium olivate, stearic acid, myristic acid, lauric acid, glyceryl stearate, sodium lauryl sulfate, sodium laureth sulfate, sodium coco sulfate, sodium cocoyl isethionate, sodium lauroyl methyl isethionate, sodium lauroyl glutamate, sodium lauroyl aspartate, polysorbate 60, polysorbate 80, lauryl hydroxysultaine, sorbeth-30 tetraoleate, sucrose laurate, polyglyceryl-10 oleate, polyglyceryl-10 dioleate, polyglyceryl-2 sesquicaprylate, and laureth-4.

12. The skin cleansing composition of claim 6, wherein the polyol comprises at least one selected from the group consisting of glycerin, 1,3-butylene glycol, dipropylene glycol, propylene glycol, polyethylene glycol, and 1,2-hexanediol.

13. The skin cleansing composition of claim 6, wherein the skin cleansing composition further comprises at least one selected from the group consisting of a moisturizer, a softener, a thickener, a preservative, a neutralizer, an acidity regulator, and a fragrance.

14. The skin cleansing composition of claim 6, which is for cleansing the face, body, or scalp.
